Europäisches Patentamt

European Patent Office

Office européen des brevets

(19)

(11) Publication number: **0 285 834 B1**

# EUROPEAN PATENT SPECIFICATION

(12)

(45) Date of publication of patent specification: **16.10.91**

(51) Int. Cl.⁵: **C07C 395/00, C30B 25/02, C30B 29/48, C23C 16/18**

(21) Application number: **88103673.5**

(22) Date of filing: **09.03.88**

(54) Allyltellurides and their use in the MOCVD growth of group II-VI epitaxial films.

(30) Priority: **07.04.87 US 35329**

(43) Date of publication of application:
**12.10.88 Bulletin 88/41**

(45) Publication of the grant of the patent:
**16.10.91 Bulletin 91/42**

(84) Designated Contracting States:
**BE CH DE FR GB IT LI NL SE**

(56) References cited:
**EP-A- 0 040 939**

**JOURNAL OF APPLIED PHYSICS, vol. 62, no. 12, 15th December 1987, pages 4929-4931, American Institute of Physics, New York, US; R.KORENSTEIN et al.: "Metalorganic growth of HgTe and CdTe at low temperatures using diallyltelluride"**

**TETRAHEDRON LETTERS, vol. 24, no. 46, 1983, pages 5109-5112, Pergamon Press Ltd., Oxford, GB; A. OSUKA et al.: "Dialkyltelluronium allylide as a novel reagent for synthesis of al-pha,beta-unsaturated epoxides"**

**APPLIED PHYSICS LETTERS, vol. 46, no. 4, 15th February 1985, pages 398-400, American Institute of Physics, New York, US; W.E. HOKE et al.: "Metalorganic growth of CdTe and HgCdTe epitaxial films at a reduced substrate temperature using diisopropyltel-luride"**

(73) Proprietor: **AMERICAN CYANAMID COMPANY**
**1937 West Main Street P.O. Box 60**
**Stamford Connecticut 06904-0060(US)**

(72) Inventor: **Valentine, Donald, Jr.**
**20 Blue Ridge**
**Ridgefield Connecticut 06877(US)**
Inventor: **Brown, Duncan William**
**30 Cobblestone Place**
**Wilton Connecticut 06897(US)**

(74) Representative: **Wächtershäuser, Günter, Dr.**
**Tal 29**
**W-8000 München 2(DE)**

## Description

The invention relates to novel tellurium compounds and their use in improved processes for making semiconductor materials comprising epitaxial growth of group II-VI semiconductor compounds.

A most useful application for semiconductor materials of group II-VI elements is the manufacture of infrared detector devices. Of special importance in this area is the preparation of epitaxial films containing cadmium, mercury and tellurium deposited on semiconductor substrates. Specific compounds that may be grown epitaxially include cadmium telluride (CdTe), mercury telluride, (HgTe) and cadmium mercury telluride (CdHgTe). Several methods have been used for the growth of epitaxial films including liquid phase epitaxy, molecular beam epitaxy, elemental vapor phase transport, and metal-organic chemical vapor deposition (MOCVD). The latter, also known as organometallic vapor phase epitaxy (OMVPE) is a most convenient and practical method for the production of high quality epitaxial films. However, this technique has not been quite successful in the case of mercury, cadmium tellurium compounds, such as for example HgCdTe. In the preparation of this material, vapors of dimethylcadmium and diethyltelluride are introduced into an MOCVD reactor and mixed with mercury vapor which is generated by heating elemental mercury. The above vapor mixture in a stream of hydrogen carrier gas is allowed to chemically interact on the surface of a crystalline substrate, such as for example CdTe, to form an epitaxial film of HgCdTe. There are several problems with this method. First, the high temperature (greater than 400°C) required to decompose the diethyltelluride causes interdiffusion of high vapor pressure metals, such as mercury, at crystal interfaces thus resulting in poor quality product. Another important detrimental effect is condensation of excess mercury vapor on the walls of the reactor unless a hot walled reactor is used. However, the hot wall reactor causes premature decomposition of the organometallic dimethylcadmium and thus formation of unwanted impurities.

In attempts to minimize these problems, Jackson in U.S. Patent No. 4,439,267 directs a stream of hydrogen carrying vapors of diethyltelluride and dimethylcadmium to a graphite susceptor having two cavities, one containing the CdTe substrate and the other liquid mercury. The susceptor is heated differentially by RF (radio frequency) to allow decomposition of the metal alkyls and vaporization of mercury, respectively, to occur at two different temperatures, such as for example 400°C and 200°C. This process is, however, quite complex and difficult to control. Furthermore, it does not eliminate condensation of excess mercury vapor elsewhere in cooler areas of the reactor. Hoke, in U.S. Patent No. 4,568,397 discloses a method in which mercury is preheated in a separate reservoir at temperatures greater than 240°C and the mercury vapor is directed to the MOCVD reactor, externally heated to greater than 240°C, and combined with the vapors of the group II organometallics such as dimethylcadmium and group VI organometallic diethyltelluride to form HgCdTe on the heated susceptor containing the substrate. However, this method does not eliminate premature decomposition of dimethylcadmium and interdiffusion is still a problem due to the high temperature (greater than 400°C) required for the decomposition of diethyltelluride. In efforts to minimize, diffusional problems associated with the high temperature growth of tellurides, less stable organotellurides such as diisopropyltelluride [Hoke, Appl. Phys. Lett. 46(4) 398 (1985)] and more recently, di-tert.-butyltelluride [Hoke, Appl. Phys. Lett. 48 (24) 1669 (1986)] have been proposed in efforts to reduce the growth temperatures. However, at these lower temperatures the growth rate for the formation of tellurides was drastically reduced. For example, CdTe an important buffer epitaxial layer for HgCdTe, which formed at the rate of 10 μm/hr. at 370°C using diisopropyltelluride, was grown at only 0.6 μm/hr. at 230°C using ditertiary butyl telluride.

In our copending United States Application Serial No. 847,370 filed April 2, 1986 we described the method for low temperature growth of groups II-VI tellurides using 2,5-dihydrotellurophene as the tellurium source. However, a need still exists for organotellurium compounds to provide low temperature epitaxial tellurides at high growth rates.

## The Invention

This invention deals with the use of certain novel allyltellurides as tellurium sources for the MOCVD epitaxial growth of group II-VI semiconductor compounds. The allyltellurides of this invention in conjunction with for example dimethylcadmium and/or dimethylmercury provide good quality epitaxial films of CdTe, HgTe, and HgCdTe. The use of dimethylmercury, as the mercury source, eliminates the need for heating the walls of the reactor in order to transport mercury vapor to the substrate, and consequently avoids premature deposition of tellurides and mercury condensation. The allyltellurides of this invention represent a class of novel compounds characterized by the presence of at least one allyl group and a lower alkyl or allyl group. The alkyl group may be one to four carbon atoms, preferably methyl. This class of compounds is represented by the formula:

$RTeCH_2CH = CH_2$ wherein R is alkyl$(C_1-C_4)$ or allyl

Examples of compounds useful in this invention include methyl allyltelluride, ethyl allyltelluride, n-propyl allyltelluride, i-propyl allyltelluride, n-butyl allyltelluride, tert. butyl allyltelluride and diallyltelluride.

The alkyl allyltellurides above may be synthesized by the reaction of a dialkylditelluride and allyl lithium or allyl Grignard reagent according to a general procedure described for the preparation of unsymmetrical dialkyl tellurides, Organometallics 2-(2)305 (1983). The starting ditelluride may in turn be prepared by the reaction of tellurium tetrachloride and an alkyl Grignard reagent. J. Organomet. Chem 255 61 (1983),

$TeCl_4$ + $RMgBr$ → $R_2Te_2$ + $R_2Te$
$R_2Te_2$ + $CH_2 = CHCH_2Li$ → $RTeCH_2CH = CH_2$ (R as defined above).

A most convenient and preferred route for the synthesis of alkyl allyltellurides is the reaction of an alkyl lithium compound first, with tellurium metal to form the lithium alkyltelluride intermediate which in turn is reacted with allyl chloride fo form the corresponding alkyl allyltelluride.

$RLi$ + $Te$ → $RTeLi$
$RTeLi$ + $CH_2 = CHCH_2Cl$ → $RTeCH_2CH = CH_2$ (R as defined above).

The diallyltelluride may be prepared by the reaction of allyl bromide or allyl chloride and sodium telluride by a procedure similar to that described for the preparation of dialkyltellurides at Naturforsch. B. Anorg. Chem. 338, 246 (1978) and at Proceedings, 3rd Int. Symp. Organoselenium and Organotellurium Compounds p. 391 (1979).

$CH_2 = CHCH_2Br$ + $Na_2Te$ → $(CH_2 = CHCH_2)_2Te$

The compounds of this invention may be used as tellurium sources for the preparation of group II-VI epitaxial films such as HgTe, CdTe, PbTe, HgCdTe, and CdMnTe. In the practice of this invention, vapors of the appropriate Group II metal such as dimethylmercury and/or dimethylcadmium are directed along with vapors of the allyltelluride derivatives of this invention, with or without premixing, at a crystalline substrate such as cadmium telluride (CdTe), indium phosphide (InP), indium antimonide (InSb), gallium arsenide (GaAs), alumina, sapphire, silicon, zirconia. In some embodiments, the group II element is added as metal vapor, e.g. mercury vapor, by diffusion, e.g., from a pool on the susceptor or by external vaporization followed by transport to the heated substrate in a carrier gas. Preferably, the substrate is in the form of a wafer which rests on a graphite susceptor heated by a ratio frequency, r.f., induction heater. A more detailed description of the apparatus may be found in J. Electrochem. Soc. Solid State Sci. Vol. 116, 1725 (1969); J. Appl. Phys. Vo.1. 54, 5087 (1983); Appl. Phys. Lett. Vol. 46, 398 (1985), Handbook on Semiconductors, Vol. 3, Chapter 5 p. 350 (1980) and U.S. Patents Nos. 4,368,098 and 4,568,397.

Using the method of this invention, epitaxial films of e.g. CdTe, HgTe and HgCdTe are successfully grown on CdTe substrate at substrate temperatures 250-350°C. The ratio of group VI organotelluride to group II compound in the gases fed to the reactor can vary widely, but preferably the mole fraction ratio will be at least 1:1. In the case in which elemental mercury is used as the group II compound, the mole fraction ratio may be as low as 0.1. More detailed description of specific embodiments of the invention are described by examples below.

## EXAMPLE 1

### PREPARATION OF METHYL ALLYLTELLURIDE

In a 1000 ml pressure bottle equipped with a stirrer is added 30.2 g. (0.237 mole) of tellurium and 400 ml tetrahydrofuran under argon atmosphere. The mixture is cooled to -78° with stirring and 175 ml of a 1.7 M ether solution of methyllithium (0.296 mole) is added over a 15 minute period and allowed to react for 45 minutes. The reaction mixture is warmed to room temperature and the tetrahydrofuran/ether solvent mixture is evaporated under vacuum.

The residue is cooled to -78° and 250 ml ether is added followed by 22.6 g (0.296 mole) allyl chloride. The reaction mixture is stirred for 18 hours and filtered. To the filtrate is added 200 ml pentane and the ether/pentane solution is washed with five-500 ml portions of deionized water and the solvent layer is separated and subjected to vacuum stripping at room temperature. The residue is distilled under vacuum at 45°/23 mm to give a total of 31.7 g (97.2 %) methyl allyltelluride.

## EXAMPLE 2

### PREPARATION OF ALKYL ALLYLTELLURIDES

Using the general procedure described in Example 1, ethyl lithium, n-propyl lithium, n-butyllithium and t-butyl lithium are reacted, respectively, with tellurium metal to form the corresponding lithium organotellurides. The latter are reacted with allyl chloride to form, respectively, ethyl allyltelluride, n-propyl allyltelluride, n-butyl allyltelluride and t-butyl allyltelluride.

## EXAMPLE 3

### PREPARATION OF DIALLYL TELLURIDE

Sodium telluride is prepared by the reaction of sodium and tellurium in liquid ammonia. After evaporation of the ammonia, a solvent, for example ether, is added and the slurry is reacted with allyl chloride to give diallyltelluride.

## EXAMPLE 4

### MOCVD GROWTH OF CdTe

Mixed cool (20°) vapors of dimethylcadmium and methyl allyltelluride (MATe) are introduced into a vertical MOCVD reactor using palladium diffused hydrogen as the carrier gas [a description of the type apparatus that may be used is found in Manasevit U.S. Patent No. 4,368,098]. The mixed vapors are directed to a crystalline cadmium telluride (CdTe) substrate placed on a graphite susceptor which is heated to 290° C by radio frequency (RF) induction. At a flow ratio of hydrogen carrier gas through dimethylcadmium to hydrogen carrier gas through MATe of 0.2, a growth rate of CdTe of approximately 3 $\mu$m/hr per 100 SCCM of hydrogen flow through the MATe bubbler is obtained. Reproducible, temperature independent CdTe growth rates as high as 24 $\mu$m/hr are achieved at or above 290° C.

## EXAMPLE 5

### MOCVD GROWTH HgTe

Following the general procedure described in Example 4, mixed cool vapors of dimethylmercury and methyl allyltelluride are reacted on a cadmium telluride substrate to form HgTe epitaxial films. Growth rates of 12 $\mu$m/hr. are achieved at 325-350°. Mercury telluride epitaxial films are also grown on CdTe substrate by the use of excess mercury vapors instead of dimethylmercury.

## EXAMPLE 6

### MOCVD GROWTH OF HgCdTe

Following the general procedure described in Example 4, mixed cool vapors of dimethylmercury, dimethylcadmium and methyl allyltelluride are allowed to impinge on indium antimonide substrate to form mercury cadmium telluride [Hg$_{1-x}$Cd$_x$Te]. Growth rates of 4 $\mu$m/hr. are achieved for x = 0.2-0.3 at 325-350° C.

## Claims

1. Organometallic tellurides of the formula:

   R - Te - CH$_2$CH = CH$_2$

   wherein R is alkyl having 1 - 4 carbon atoms, or allyl.

2. A compound defined by Claim 1 wherein R is methyl.

3. A compound defined by Claim 1 wherein R is allyl.

4. A process for the preparation of group II-VI epitaxial films which comprises directing vapors of at least one group II metal or organometallic compound of a group II metal along with vapors of an organotellurium compound at a crystalline substrate in a MOCVD reactor with at least the substrate heated to temperature sufficient for growth of an epitaxial film of a group II tellurium compound by reaction of said vapors on the substrate characterized by the fact that the organotellurium compound is a telluride of the formula:

   R - Te - CH$_2$CH = CH$_2$

   wherein R is alkyl having 1 - 4 carbon atoms or allyl.

5. A process as defined by Claim 4 wherein the substrate is heated to temperature in the range from 250° to 350°.

6. A process as defined by Claim 4 wherein the mole ratios of the telluride vapor to the group II organometallic vapor fed to the reactor is at least 1:1.

7. A process defined by Claim 4 wherein the organotellurium compound is methyl allyltelluride.

8. A process as defined by Claim 4 wherein the group II organometallic compound is dimethylmercury or dimethylcadmium or both.

9. A process as defined by Claim 4 wherein the mole ratios of group II metal vapor to the telluride vapor fed to the reactor is 1:1 to 10:1.

10. A process as defined by Claim 9 wherein the group II metal vapor is mercury vapor.

11. A process defined by Claim 6 wherein the organotellurium compound is methyl allyltelluride.

## Revendications

1. Tellurures organométalliques de formule :

R - Te - CH₂CH = CH₂

dans laquelle R représente un groupe alkyle comportant de 1 à 4 atomes de carbone ou un groupe allyle.

2. Composé selon la revendication 1, R représentant un groupe méthyle.

3. Composé selon la revendication 1, R représentant un groupe allyle.

4. Procédé de préparation de films épitaxiaux dérivés d'éléments des groupes II et VI, selon lequel on dirige des vapeurs d'au moins un métal du groupe II ou d'un composé organométallique dérivé d'un métal du groupe II, en combinaison avec des vapeurs d'un composé organotellurique, sur un substrat cristallin dans un réacteur de MOCVD, le substrat étant au moins chauffé à une température suffisante pour permettre la croissance d'un film épitaxial d'un composé dérivé du tellure et d'un élément du groupe II, par réaction de ces vapeurs sur le substrat, caractérisé en ce que le composé organotellurique, est un tellurure de formule :

R - Te - CH₂CH = CH₂

dans laquelle R représente un groupe alkyle comportant de 1 à 4 atomes de carbone, ou un groupe allyle.

5. Procédé selon la revendication 4, dans lequel le substrat est chauffé à une température de 250 à 350 °C.

6. Procédé selon la revendication 4, dans lequel les rapports molaires de la vapeur de tellurure à la vapeur organométallique à base d'élément du groupe II, introduites dans le réacteur, est d'au moins 1:1.

7. Procédé selon la revendication 4, dans lequel le composé organotellurique, est le tellurure de méthyle et d'allyle.

8. Procédé selon la revendication 4, dans lequel le composé organométallique dérivé d'élément du groupe II, est le diméthylmercure, le diméthylcadmium ou les deux.

9. Procédé selon la revendication 4, dans lequel les rapports molaires de la vapeur de métal du groupe II à la vapeur de tellurure introduites dans le réacteur, est de 1:1 à 10:1.

10. Procédé selon la revendication 9, dans lequel la vapeur de métal du groupe II, est de la vapeur de mercure.

11. Procédé selon la revendication 6, dans lequel le composé organotellurique, est le tellurure de méthyle et d'allyle.

## Patentansprüche

1. Organometallische Telluride der Formel:

R - Te - CH₂CH = CH₂

wobei R für Alkyl mit 1 bis 4 Kohlenstoffatomen oder Allyl steht.

2. Verbindung gemäß Anspruch 1, wobei R für Methyl steht.

3. Verbindung gemäß Anspruch 1, wobei R für Allyl steht.

4. Verfahren zur Herstellung von Gruppe II-VI-Epitaxie-Filmen, umfassend das Richten von Dämpfen von mindestens einem Gruppe II-Metall oder organometallischer Verbindung von einem Gruppe II-Metall gemeinsam mit Dämpfen einer Organotellurverbindung auf ein kristallines Substrat in einem MOCVD-Reaktor, wobei mindestens das Substrat auf eine Temperatur erhitzt ist, die ausreicht für das Wachstum eines Epitaxie-Films von einer Gruppe II-Tellurverbindung durch Umsetzung der Dämpfe auf dem Substrat, dadurch gekennzeichnet, daß die Organotellurverbindung ein Tellurid der folgenden Formel ist:

R - Te - CH₂CH = CH₂

wobei R für Alkyl mit 1 bis 4 Kohlenstoffatomen oder Allyl steht.

5. Verfahren gemäß Anspruch 4, wobei das Substrat auf eine Temperatur im Bereich von 250° bis 350° C erhitzt ist.

6. Verfahren gemäß Anspruch 4, wobei die Molverhältnisse von dem Telluriddampf zu dem Gruppe II-organometallischem Dampf, die in den Reaktor eingespeist werden, bei mindestens 1:1 liegen.

7. Verfahren gemäß Anspruch 4, wobei die Organotellurverbindung Methylallyltellurid ist.

8. Verfahren gemäß Anspruch 4, wobei es sich bei der Gruppe II-Organometallverbindung um Dimethylquecksilber oder Dimethylcadmium oder beide handelt.

9. Verfahren gemäß Anspruch 4, wobei die Molverhältnisse von in den Reaktor eingespeistem Gruppe II-Metalldampf zu dem Telluriddampf 1:1 bis 10:1 betragen.

10. Verfahren gemäß Anspruch 9, wobei der Gruppe II-Metalldampf Quecksilberdampf ist.

11. Verfahren gemäß Anspruch 6, wobei die Organotellurverbindung Methylallyltellurid ist.